Europäisches Patentamt

European Patent Office

Office européen des brevets

(19)

(11) Numéro de publication: **0 197 889
B1**

(12) **FASCICULE DE BREVET EUROPEEN**

(45) Date de publication du fascicule du brevet:
23.05.90

(51) Int. Cl.⁵: **A61N 1/36**

(21) Numéro de dépôt: **86810143.7**

(22) Date de dépôt: **26.03.86**

(54) **Dispositif de stimulation neuro-musculaire électrique.**

(30) Priorité: **03.04.85 CH 1438/85**

(43) Date de publication de la demande:
**15.10.86 Bulletin 86/42**

(45) Mention de la délivrance du brevet:
**23.05.90 Bulletin 90/21**

(84) Etats contractants désignés:
**AT BE CH DE FR GB IT LI LU NL SE**

(56) Documents cités:
**EP-A- 0 087 617
EP-A- 0 165 049
WO-A-83/02901
DE-A- 1 764 091
DE-A- 2 903 392
FR-A- 2 155 939
FR-A- 2 528 709
US-A- 4 505 275**

**ENGINEERING IN MEDICINE AND BIOLOGY, IEEE,
septembre 1983, pages 29-37, New-York, US; J.
THOMAS MORTIMER: "Applied neural control, part 2"**

(73) Titulaire: **MEDICOMPEX S.A., c/o BBC Sécheron
S.A. 14, avenue de Sécheron, CH-1202 Genève(CH)**

(72) Inventeur: **Brodard, Roland, Avenue des Châtaigniers, 4,
CH-1844 Villeneuve(CH)**

(74) Mandataire: **Kirker, Gaylord Emile et al, c/o KIRKER &
Cie S.A. 14, Rue du Mont-Blanc Case postale 872,
CH-1211 Genève 1(CH)**

## Description

Domaine de l'invention

On connaît depuis longtemps des appareils de stimulation neuro-musculaire électrique utilisés soit pour éviter une atrophie musculaire après un accident ou une opération, soit pour faire travailler certains muscles afin de les fortifier. Beaucoup de ces appareils utilisent des trains d'impulsions électriques variant considérablement d'un appareil à l'autre. La fréquence des impulsions est en général choisie dans la gamme allant de l'ordre du Hz à celui des dizaines de KHz.

Des études expérimentales de physiologie effectuées au cours de ces dernières années ont mis en évidence le fait que le travail des muscles est beaucoup plus complexe qu'on ne l'avait imaginé auparavant et que, par conséquent, les dispositifs de stimulation neuro-musculaire électrique proposés jusqu'ici sont en fait assez rudimentaires et mal adaptés à la physiologie des muscles. On trouve actuellement dans la littérature des données éparses à ce sujet, qui montrent que le tissu musculaire n'est pas homogène et qu'il est formé d'au moins trois types de fibres: une première catégorie dite de fibres à fonctionnement lent et deux types de fibres à fonctionnement rapide, respectivement très rapide. La stimulation de ces trois types de fibres musculaires ne peut pas se faire par les mêmes fréquences de courant stimulateur; certaines fréquences ne conviennent qu'aux fibres lentes et d'autres fréquences qu'aux fibres rapides.

Par ailleurs, d'autres études ont montré que les traitements de relativement courte durée appliqués jusqu'ici sont tout à fait insuffisants pour lutter contre l'atrophie musculaire et pour assurer un développement musculaire, par exemple pour des athlètes. Il faut en réalité envisager des traitements quotidiens de plusieurs heures par jour, jusqu'à 5 heures par exemple. Mais ceci pose des problèmes, car d'autres études encore ont montré que des traitements de longue durée à fréquences constantes risquent de produire des effets nocifs dans les muscles, soit par détérioration des fibres, soit par modification des fibres, avec pour résultat que des fibres lentes se transforment, en cours de traitement, en fibres rapides, ou inversement, selon la fréquence de stimulation utilisée. En effet, on sait depuis peu que les fibres musculaires n'ont pas un caractère immuable. Au contraire, la proportion des trois types de fibres peut se modifier en fonction du travail - donc de l'excitation - imposé au muscle. Les fibres sont capables de se transformer d'une catégorie dans une autre. Il convient donc de choisir de façon appropriée les fréquences et d'utiliser les différentes fréquences de manière équilibrée pour stimuler les fibres lentes et les fibres rapides d'une façon elle-même équilibrée, afin de ne pas introduire de perturbations ultérieures dans la structure des muscles.

D'autres études encore ont montré que, surtout en ce qui concerne les fibres rapides, il faut absolument respecter entre les différents trains d'ondes de stimulation électrique, des temps de repos beaucoup plus longs qu'on ne l'avait imaginé auparavant, afin d'assurer que le muscle ne soit pas à la longue intoxiqué par les corps chimiques produits par le travail musculaire lui-même. En d'autres termes, il faut laisser entre les trains d'impulsions successifs un temps de réparation suffisant et ce temps est beaucoup plus long qu'on ne l'avait imaginé.

Un autre problème encore se pose du fait de la longueur des traitements nécessaires, comme on vient de le voir. Il n'est pas concevable, pour des raisons économiques, de faire faire au patient, en milieu hospitalier ou chez le physiothérapeute, des séances quotidiennes de plusieurs heures, qui immobiliseraient des locaux, du personnel, des appareils coûteux et nombreux, etc., aussi convient-il d'envisager d'avoir pour chaque patient un appareil lui permettant d'effectuer un traitement d'après un programme établi sur mesures pour lui, selon son cas. Mais bien entendu un tel appareil devrait être simplifié au maximum et être piloté par un programme enregistré sur un élément de transfert, programme qui, lui, pourrait alors être établi en milieu hospitalier ou par le thérapeute sur un appareil programmeur qui servirait à établir le programme en liaison avec un appareil stimulateur du patient branché sur celui-ci et permettant au médecin ou au thérapeute de régler les fréquences, les durées, les intervalles entre trains d'ondes, etc., bref les différents paramètres de manière adaptée au cas, de limiter les intensités maxima au-dessous des seuils dangereux, cet appareil permettant une fois toutes les données ainsi établies sous contrôle du médecin qui observe le patient, d'enregistrer sur un support, une carte magnétique par exemple, tout programme qui, lui, sera alors ultérieurement introduit dans l'appareil stimulateur du patient qui, dès lors, travaillera de façon autonome.

Lors de cette programmation, il convient évidemment de limiter l'intensité de courant utilisée pour les impulsions, afin de ne pas créer de dommages dans les tissus. Mais il faut, de plus, prévoir des moyens grâce auxquels le réglage de l'intensité ne puisse avoir lieu que durant la phase de travail des trains d'impulsions. En effet, si ce réglage a lieu durant la phase d'établissement d'un train d'impulsions ou durant le temps d'extinction de l'impulsion ou encore durant la période de repos entre deux trains d'impulsions, le thérapeute n'a plus le contrôle des effets de son réglage et cela peut être dangereux.

Ces moyens de sécurité sont opérants aussi lorsque le patient modifie lui-même l'intensité du courant de stimulation, en agissant sur les boutons 25.

Etat de la technique

On a déjà proposé, dans le brevet français No 1.213.080, d'employer un appareil programmeur pour enregistrer sur un support un programme de traitement et un appareil d'application au patient commandé par le programme enregistré sur ce support. Ainsi on peut, avec un seul appareil programmeur, avoir un nombre quelconque d'appareils d'application munis chacun d'un programme adapté au cas du patient auquel cet appareil d'application est confié pour traitement à domicile. Dans ce brevet, il est prévu que la programmation, pour un patient donné, se fait pendant que le patient est soumis au traitement à titre de test guidant le programmeur.

Dans la demande de brevet allemande publiée DOS 29.03.392, il est proposé d'effectuer la stimulation au moyen d'un appareil commandé par un programme préalablement enregistré sur un support placé dans cet appareil, programme choisi en fonction du cas du patient.

Dans la demande de brevet européen publiée No 0.087.617, il est décrit un appareil programmeur, pour enregistrer sur un support un programme de traitement adapté au cas de chaque patient, et un appareil d'utilisation dans lequel on introduit ce support et qui est commandé par le programme.

La demande de brevet européen publiée EP-A 0 087 617 décrit aussi un appareil pour le traitement décentralisé qui a la particularité que les paramètres, pour chaque traitement individuel, sont choisis et fixés par le thérapeute et ne peuvent pas être modifiés par le patient.

La demande de brevet français publiée No 2.528.709 (dérivant des demandes de brevets USA No 390.026 et 390.027) décrit un stimulateur électrique biologique destiné à être porté par un patient (traitement ambulant) et qui est commandé par un support d'enregistrement constitué par une carte de programme insérée dans le stimulateur et programmée en fonction du cas du patient. Cet appareil est décrit comme analgésique.

Aucun des appareils décrits dans ces publications n'est conçu pour tenir compte des conditions rappelées plus haut et qu'il est nécessaire de respecter pour obtenir une rééducation musculaire ou un développement musculaire efficace et sans danger d'endommagement des tissus musculaires.

La présente invention vise à fournir un dispositif de stimulation neuro-musculaire électrique agencé pour satisfaire aux conditions que les recherches physiologiques récentes ont montré être nécessaires pour assurer un traitement efficace des muscles exempt de risque d'endommagement de ceux-ci. Elle a pour objet un dispositif de stimulation neuro-musculaire électrique pour provoquer des contractions et un exercice ou un entraînement musculaires, comprenant des caractéristiques définies dans la revendication 1.

Description des dessins

Les dessins annexés représentent, à titre d'exemple, une forme d'exécution du dispositif objet de l'invention.

Fig. 1 est une vue schématique, en perspective, de l'ensemble de cette forme d'exécution, qui comprend un élément programmeur A et un élément stimulateur B reliés par un câble amovible.

Fig. 2 est un schéma bloc de l'élément programmeur A de la fig. 1.

Fig. 3 illustre, sous forme d'un schéma bloc, une carte de programme utilisée avec le dispositif selon fig. 1.

Fig. 4 est un schéma bloc de l'élément stimulateur B de la fig. 1.

Fig. 5 illustre l'allure de la courbe de la variation de la grandeur de la force de contraction musculaire, en fonction de l'augmentation de l'intensité du signal impulsionnel de stimulation du muscle.

Fig. 6 est un diagramme schématique de l'amplitude en fonction du temps du signal impulsionnel délivré à la sortie du dispositif selon fig. 1, et qui met en évidence les zones dans lesquelles l'augmentation de la force de contraction (pendant la programmation) peut être autorisée, et celles dans lesquelles cette dernière doit au contraire être interdite par le dispositif, comme il est expliqué plus loin.

Fig. 7a, 7b, 7c illustrent schématiquement des formes d'ondes d'impulsions usuelles, programmables à choix.

Fig. 8a, 8b montrent schématiquement l'application des impulsions en mode continu et en mode intermittent (trains d'impulsions).

Fig. 9a, 9b illustrent le mode de travail absolu des sorties et le délai de réciprocité en mode alterné.

Fig. 10 est un diagramme de la durée de la période de travail, de son temps d'établissement et d'extinction, ainsi que de la durée de la période de repos.

Description de l'invention

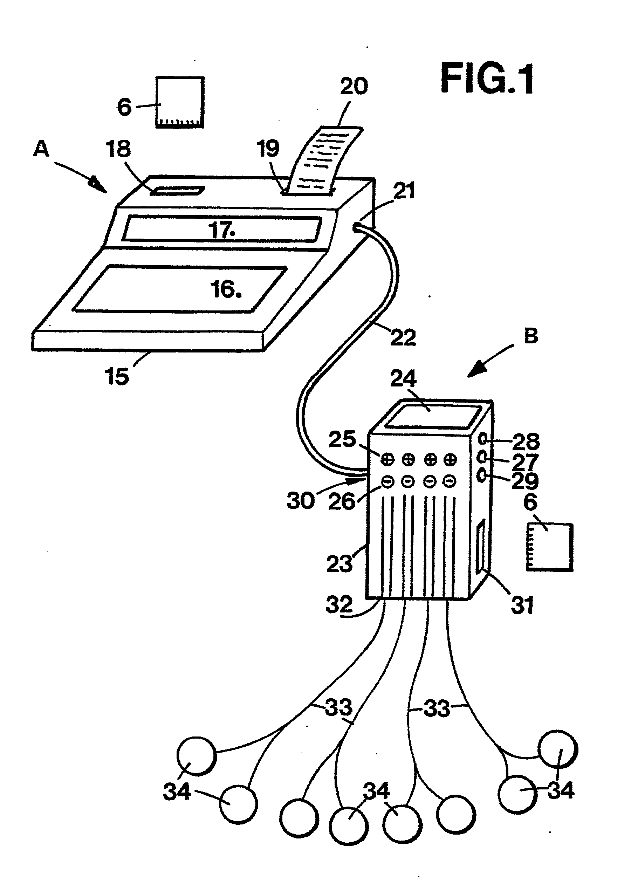

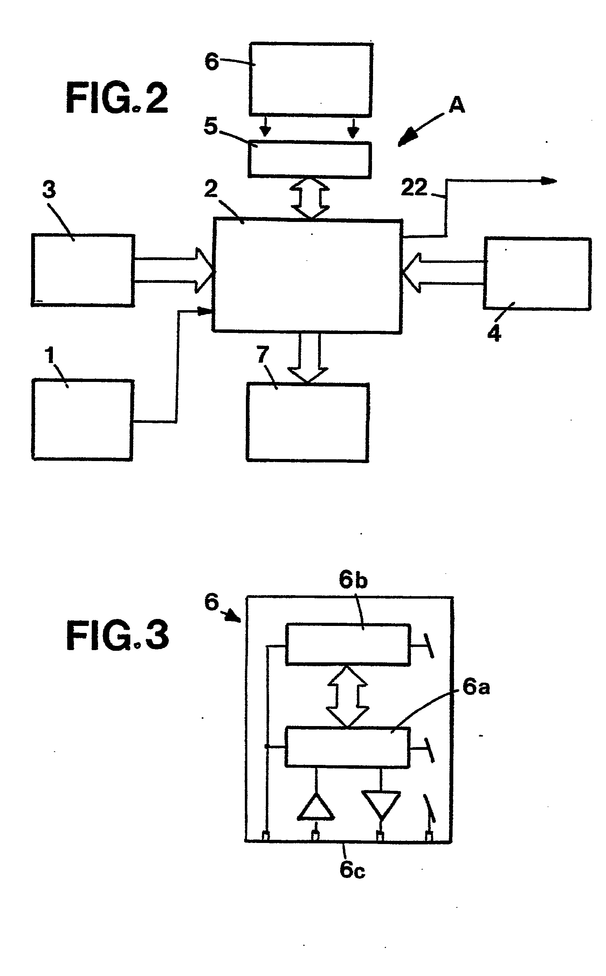

Le dispositif illustré sur la fig. 1 comprend un élément programmeur A, portatif et autonome, qui consiste en un boîtier 15 contenant les parties constitutives, mécaniques et électroniques, conformes au schéma bloc illustré par la fig. 2; tandis que l'extérieur dudit boîtier met à disposition de l'opérateur: un clavier de commande et d'entrée des données situé en 16; un écran d'affichage LCD situé en 17; une ouverture 18 permettant l'insertion d'une carte programme 6; une ouverture de livraison 19 d'étiquettes 20 éditées par une mini-imprimante incorporée dans le bâti; ainsi qu'un connecteur 21 permettant la jonction série avec un élément stimulateur B, au moyen d'un câble détachable 22. Ces étiquettes indiquent en clair le programme enregistré sur la carte programme 6 introduite en 18.

L'élément stimulateur B est portatif et autonome; il est exécuté selon un concept général du style "walkman". Son boîtier 23 contient les parties constitutives, mécaniques et électroniques, conformes au schéma bloc de la figure 3; tandis que l'extérieur dudit boîtier met à disposition de l'utilisateur: un écran d'affichage LCD 24, permettant le contrôle des fonctions (durée du traitement, séquence en cours, état de charge des batteries et bargraphes indiquant l'intensité du signal de stimulation de chaque sortie); une commande permettant d'augmenter 25 ou de diminuer 26 l'intensité du signal de stimulation de chaque sortie; une commande de démarrage du traitement 27; une commande d'interruption momentanée dudit traitement 28; une commande de suppression momentanée de la période de repos 29; un connecteur 30 permettant de raccorder le câble détachable 22 assurant la jonction série avec le programmeur 15; une ouverture d'insertion 31 pour la carte programme 6; et des connecteurs 32 prévus pour le raccordement des câbles 33 de chaque paire d'électrodes 34.

Sur les fig. 1 et 2, A est un élément programmeur.

Il inclut une batterie rechargeable 1 alimentant un microcontrôleur 2 comprenant un microprocesseur, auquel sont directement intégrées une mémoire ROM (read only memory) et une mémoire RAM (random access memory). Ce microcontrôleur permet l'enregistrement, la mémorisation et la gestion de tous les paramètres d'excitation et de contrôle du déroulement d'un traitement, conformément à une programmation préalablement mémorisée. Les principaux paramètres d'excitation sont les suivants:
- La forme de l'impulsion, soit la variation de son amplitude en fonction de sa durée.
- L'amplitude de l'impulsion.
- La durée de l'impulsion.
- La fréquence de répétition des impulsions dans le temps.
- La durée d'un train d'impulsions.
- La durée de la pause qui sépare deux trains d'impulsions consécutifs.
- Le temps ou la pente d'établissement d'un train d'impulsions.

Le microcontrôleur 2 est relié au clavier 3 placé en 16 (fig. 1) et permettant à l'opérateur de sélectionner lesdits paramètres d'excitation et de contrôle du déroulement du traitement et de déterminer la valeur attribuée à chacun des paramètres sélectionnés, puis d'introduire ces données dans le microcontrôleur où elles seront gérées conformément au programme préalablement établi.

Le clavier 3 et le microcontrôleur 2 sont reliés à l'affichage alphanumérique 4, du type écran LCD (liquid crystal display) qui permet le dialogue opérateur-machine et le contrôle des données introduites. Cet affichage est situé en 17 sur la fig. 1.

Le microcontrôleur est également relié à un connecteur 5, moyen de liaison avec un support amovible de données 6 (fig. 1 et 3), représenté en l'occurrence par une carte en matière plastique, par exemple du format carte de crédit standard, et qui contient (fig. 3) un microprocesseur 6a, relié à une mémoire EEPROM (electrical erasable programmable read only memory) 6b et à un connecteur 6c. Ledit support 6 se distingue par sa mémoire non volatile et par sa faculté d'écriture et de lecture électrique dans données mémorisées, comme on le verra plus loin.

Le microcontrôleur 2 est encore relié à une mini-imprimante 7 qui permet l'édition sur une étiquette autocollante 20, des données qui caractérisent le traitement programmé de stimulation neuro-musculaire prescrit, enregistré sur la carte 6 grâce au clavier 3 (16), et le patient auquel il s'adresse. Lesdites données peuvent être introduites par le clavier 3 et/ou provenir d'une programmation de gestion du traitement préalablement mémorisée dans le microcontrôleur 2. L'étiquette auto-collante est ensuite appliquée à la carte-support de données programmable, dont elle permettra l'identification du contenu en langage clair.

Enfin, le microcontrôleur 2, par le moyen du connecteur et d'un câble 22 (fig. 1), est doté d'une jonction série permettant une fonction on-line de l'élément programmeur autonome A illustré aux figures 1 et 2 avec un élément stimulateur neuro-musculaire B.

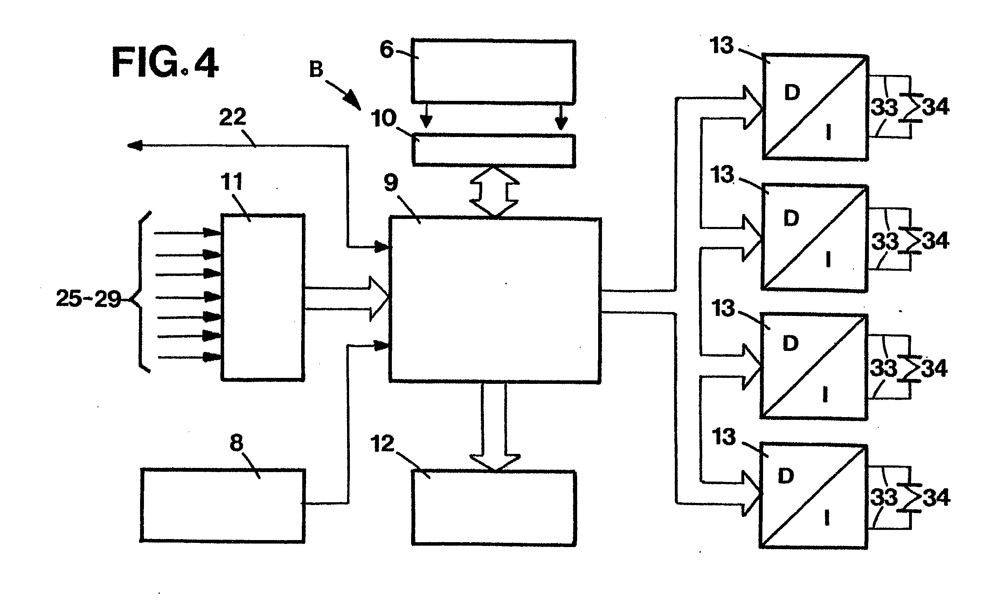

L'élément stimulateur B (fig. 1 et 4) inclut une batterie électrique 8, de préférence un block amovible de batteries nickel-cadmium rechargeables, alimentant un microcontrôleur 9. Ce microcontrôleur est relié au moyen du connecteur 10 au support amovible de données, en l'occurrence représenté par la carte programmable 6, déjà décrite plus haut et illustrée par les fig. 1 et 3.

Les microcontrôleurs, respectivement 9 de l'élément stimulateur B et 6a du support amovible de données 6, ont alors la faculté de communiquer entre eux de manière interactive, permettant ainsi non seulement la gestion de toutes les données nécessaires au déroulement correct d'un programme de traitement, mais également un fonctionnement on-line du programmeur et du stimulateur quand ces deux éléments du dispositif sont reliés l'un à l'autre par la jonction série 22 (fig. 1).

Par fonction on-line, on entend que tous les paramètres traités par le programmeur peuvent être testés en temps réel sur le patient, tels qu'ils sont introduits et restitués par le stimulateur, et tels qu'ils doivent être intégrés au programme qu'ils contribuent à définir. Dans le même temps, chaque paramètre est mémorisé sur le support amovible de données 6. Il suffit dès lors d'interrompre le fonctionnement on-line pour que ledit support amovible de données soit chargé du programme testé en temps réel sur le patient.

L'ensemble qui vient d'être décrit permet de charger la carte programme 6 de plusieurs façons:

La carte programme étant insérée dans le stimulateur, elle se charge simultanément à l'établissement du programme de traitement fait au moyen du programmeur et à son test on-line sur le patient au moyen du stimulateur.

La carte programme 6 étant insérée en 18 dans le programmeur A, elle peut alors être chargée au moyen dudit programmeur en l'absence du stimulateur B, mais sans que le programme établi puisse être simultanément testé sur le patient.

Une carte programme 6 insérée dans le programmeur A peut être lue et mémorisée, puis remplacée en 18 par une autre carte; elle peut alors être copiée sur cette dernière, permettant ainsi la duplication d'un programme sur une ou plusieurs cartes programmables.

La communication interactive entre les microcontrôleurs, respectivement du stimulateur et de la carte amovible support de données, permet l'écriture et la mémorisation sur cette dernière d'informations relatives au déroulement réel du traitement programmé.

Ainsi, à titre d'exemple, il peut être prévu d'enregistrer la durée d'application réelle effectuée par le patient, du traitement qui lui a été prescrit. Les données réelles ainsi restituées par le stimulateur et mémorisées sur la carte amovible support de données, peuvent être interprétées et relues au moyen du programmeur. Le thérapeute dispose ainsi d'un précieux moyen de contrôle de l'observance de l'application du traitement prescrit.

En référence à la figure 4, le microcontrôleur 9 est aussi relié, au moyen d'un circuit interface d'entrée 11, aux organes de commande manuelles du stimulateur, seuls accessibles au patient et qui, dans l'exemple, se résument à:
- Une commande 28 de mise en marche et d'arrêt (on/off) du stimulateur.
- Une commande 29 de suppression momentanée de la période de repos entre deux périodes de contraction consécutives, dans le but de faciliter le réglage de la grandeur de la force de la contraction musculaire engendrée par la stimulation.
- Une commande 27 de démarrage du début du traitement, d'interruption momentanée du traitement, ou de reprise après une interruption, ou encore de démarrage d'une séquence non automatique.
- Une commande de réglage de l'amplitude du signal impulsionnel délivré à chacune des sorties; les boutons 25 correspondent à des augmentations d'amplitude et les boutons 26 à des diminutions. Ce sont des interrupteurs actifs durant la pression sur eux.

Le microcontrôleur 9 est encore relié à un affichage alphanumérique 12 du type écran LCD (liquid crystal display) visible en 24 sur la fig. 1 et qui indique la durée résiduelle du traitement encore à effectuer, l'identification de la séquence en cours d'exécution, lè niveau de charge des batteries, et au moyen de bargraphes, l'amplitude, sous forme analogique, du signal impulsionnel délivré à chacune des sorties.

Le microcontrôleur 9 est enfin relié à chacun des étages de sortie 13. Chaque étage de sortie, isolé galvaniquement l'un par rapport à l'autre, assure, au moyen d'un convertisseur D/I, la transformation du signal digital programmé, généré par le microcontrôleur, en un signal impulsionnel conforme, délivré par ladite sortie. Ce signal est appliqué au patient par voie transcutanée, au moyen des câbles 33 et des paires d'électrodes de surface 34, au nombre d'une paire, par sortie.

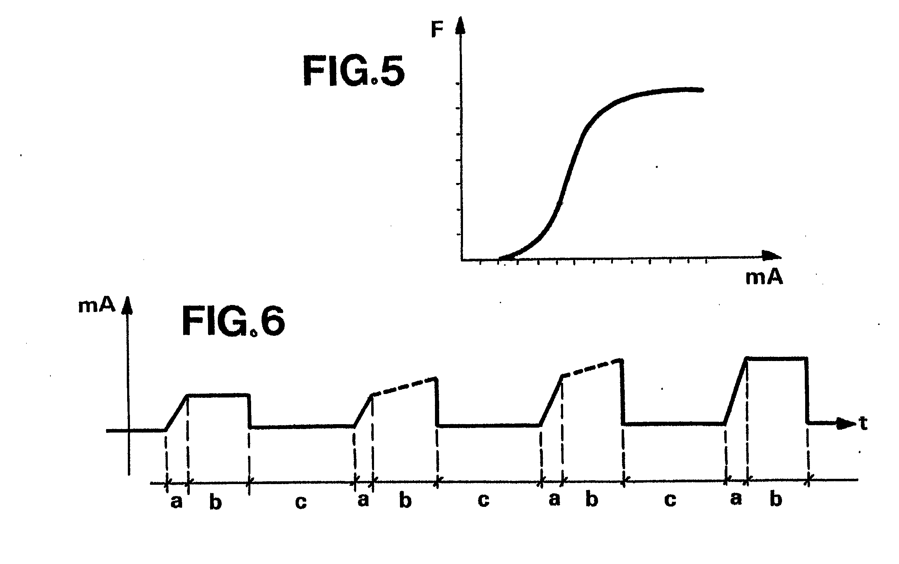

L'augmentation de la grandeur de la force délivrée par la contraction musculaire, en fonction de l'augmentation de l'intensité du signal impulsionnel de stimulation, n'est ni linéaire, ni logarithmique. Elle dessine une courbe caractéristique, propre à la réaction du muscle soumis au passage du courant de stimulation, telle qu'elle est illustrée à la figure 5. Par conséquent, il s'avère impossible de réaliser par les moyens usuels un réglage de la force de contraction musculaire qui soit à la fois commode, régulièrement progressif, fin et précis; ces moyens sont généralement représentés par des potentiomètres linéaires ou logarithmiques. Un progrès considérable consiste à attribuer au microcontrôleur 2, le contrôle intégral de la variation de l'intensité du signal impulsionnel délivré à la sortie, tant en ce qui concerne la détermination de sa forme que de son mode d'application. Par une programmation adéquate dudit microcontrôleur, il est possible d'adapter la variation de l'intensité du signal impulsionnel de sortie en fonction du temps 6, de telle sorte que la variation correspondante de la grandeur de la force de contraction F remplisse les conditions optimales de réglage souhaitées pour assurer la sécurité du patient (fig. 5).

Ladite programmation définit également le mode d'application grâce au microcontrôleur 9, en ce sens qu'elle n'autorise une augmentation de la force de contraction que pendant une période de travail (force de contraction établie en plateau), et l'interdit au contraire pendant le temps d'établissement de ladite force, ainsi qu'en l'absence de stimulation, par exemple durant une période de repos entre deux contractions consécutives.

Sur la fig. 6, qui représente l'amplitude, en milliampères, des impulsions électriques de stimulation, en fonction du temps 6, on a désigné par a, b, et c:
a = durée d'établissement de l'impulsion
b = intensité nominale de plateau
a + b = durée du train d'impulsions = période de travail musculaire
c = période de repos.

Le réglage de l'intensité n'est autorisé que pendant la période b, car c'est au cours de celle-ci seulement que le programmeur peut observer sur le patient l'effet de ce réglage. Le microcontrôleur 9 interdit (empêche) le réglage de se faire pendant les périodes a et c, pour assurer la sécurité du patient. Il s'agit là d'une mesure de sécurité d'une extrême importance, car toute augmentation inattendue et non contrôlée in vivo en temps réel de la force de contraction musculaire peut avoir des conséquences nocives et dangereuses pour le muscle.

La mise en oeuvre de ce programme de réglage de la force de contraction est pilotée par les organes de commande manuelle 25, 26 à action momentanée. Lorsque l'opérateur maintient le premier en position active, la force de la contraction augmente progressivement, conformément à la loi de variation programmée, tandis que l'action du second entraîne la diminution de cette même force.

La méthode destinée à déterminer correctement les différents paramètres optimaux d'excitation et de contrôle du déroulement d'un traitement sort du cadre de la présente invention. Car ces paramètres varient grandement en fonction du schéma d'activité particulier que le thérapeute veut imposer aux muscles, en accord avec ses objectifs thérapeutiques spécifiques à chaque cas et à chaque patient, ainsi qu'avec la physiologie moderne du muscle.

Cependant, à titre d'exemple, les paramètres programmables d'excitation et de contrôle du déroulement d'un traitement sont indiqués avec l'étendue des valeurs programmables qui peuvent leur être usellement attribuées:

Durée d'une séance de traitement:
- exprimée en temps: de 1 minute à 10 heures, résolution 1 minute;
- exprimée en nombre de cycles de travail: de 1 à 100.

Nombre de séquences, c'est-à-dire temps pendant lequel les paramètres d'excitation programmés restent constants) comprises dans une séance de traitement: de 1 à 10.

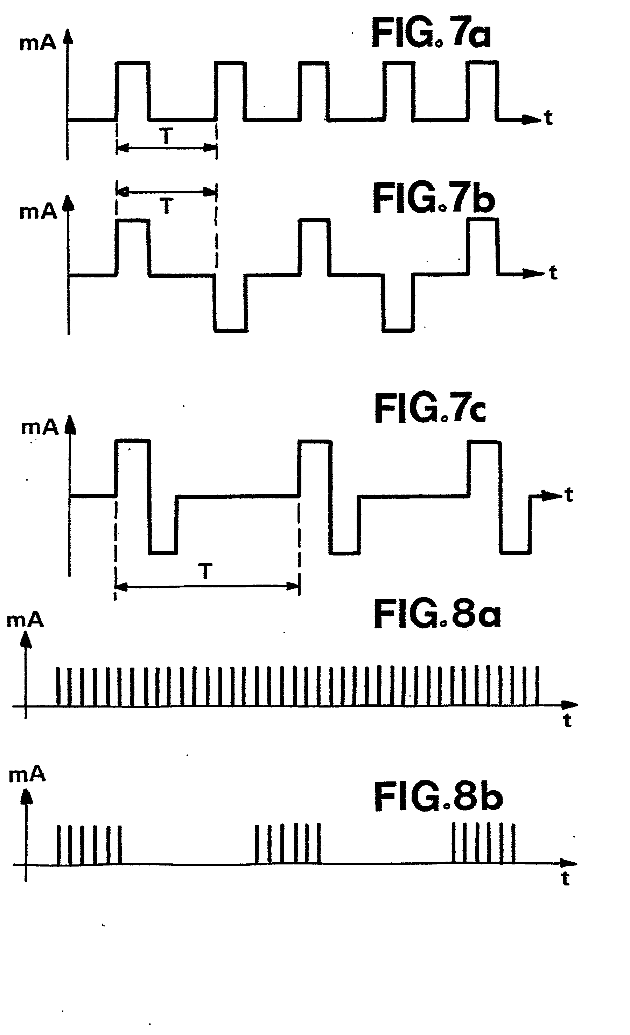

Puis à l'intérieur de chaque séquence:
Durée de la séquence:
- inférieure ou égale à la durée du traitement.
Impulsion ou stimulus (forme d'onde rectangulaire de période T):
- type monophasique (unidirectionnel), fig. 7a
- type biphasique à alternance symétrique, fig. 7b
- type biphasique à symétrie directe, fig. 7c.

Durée de l'impulsion: de 0,01 à 1,0 milliseconde, résolution 10 us.

Fréquence de répétition des impulsions: de 1 à 200 impulsions par seconde.

Mode d'application des impulsions:
- permanent continu, fig. 8a
- intermittent cyclique (succession de périodes de travail et de repos), fig. 8b.

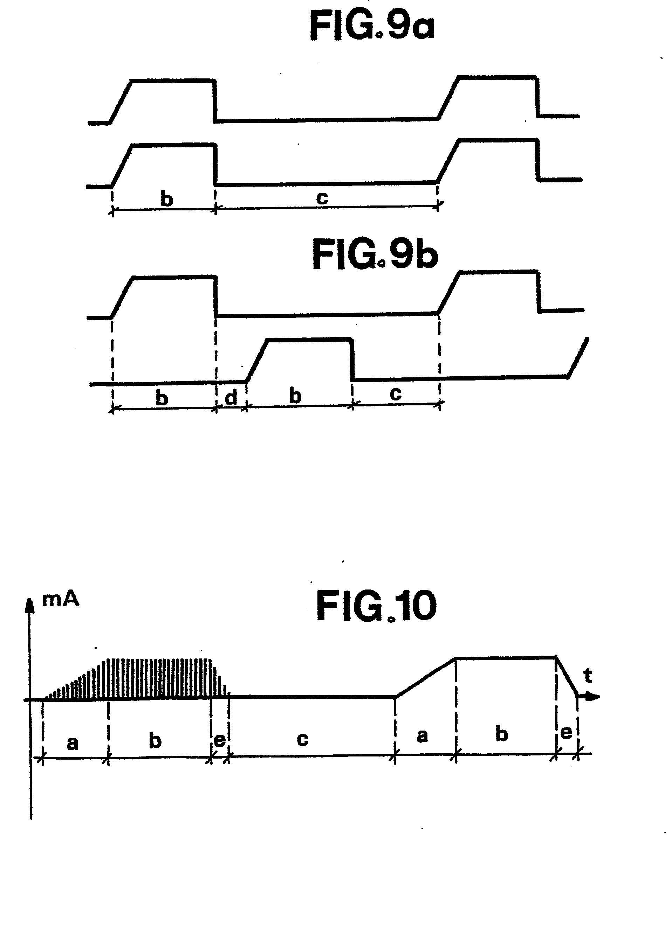

Mode de travail absolu des sorties:
- synchrone: toutes les sorties travaillent simultanément, fig. 9a
- alterné: une moitié des sorties disponibles travaille en alternance avec l'autre moitié (fig. 9b)
b = période de travail
c = période de repos
d = période de réciprocité (décalage entre les impulsions alternées des sorties).

Commande de la délivrance des impulsions aux sorties:
- automatique, conforme à la programmation du mode d'application des impulsions;
- par commande manuelle externe (remote control), qui déclenche une phase du mode d'application programmé.

La fig. 10 représente l'intensité en mA de l'excitation, en fonction du temps. Les périodes a, b, et c sont les mêmes que sur la fig. 6. On a désigné par e le temps d'extinction d'un train d'impulsions.

Dans ce cas, on peut avoir:

Durée de la période de travail (a + b + e) = contraction = 1 à 60 secondes; résolution 1 seconde.

Temps (a) d'établissement de la période de travail: de 1 à 20 secondes, résolution 1 seconde.

Temps d'extinction (e) de la période de travail: de 1 à 20 secondes, résolution 1 seconde.

Délai de réciprocité (d) en mode alterné (fig. 9b): de 10 à 1000 millisecondes, résolution 10 ms.

Durée de la période de repos (c) (relâchement musculaire) : de 1 à 200 secondes, résolution 1 seconde.

Commutation d'une séquence à l'autre:
- automatique
- manuelle.

Le dispositif décrit présente les avantages suivants:

Il est possible de disposer d'une série de cartes programmes telle que 6, corresopndant à divers programmes standards et de les utiliser comme base de programmation individuelle, en modifiant leur programme au moyen du clavier, pour faire l'adaptation particulière à chaque patient.

L'élément de programmation A peut aussi être utilisé par le thérapeute pour consulter les cartes de renseignements que l'on introduirait en 18 et dont il pourrait lire le contenu en 17.

Les moyens générateurs de trains d'impulsions du stimulateur incluent les moyens de soumettre la commande manuelle de réglage de l'intensité du signal délivré à une sortie au contrôle du microcontrôleur 9. Par une programmation adéquate dudit microcontrôleur, il est alors possible d'adapter automatiquement la variation de l'intensité dudit signal en fonction du temps, de telle sorte que la variation correspondante de la grandeur de la force de la contraction musculaire provoquée par ledit signal remplisse les conditions optimales permettant un réglage commode, régulièrement progressif, fin et précis.

Les moyens de contrôle de la commande manuelle de réglage de l'intensité du signal impulsionnel délivré à une sortie incluent les moyens de n'autoriser une augmentation dudit signal et par conséquent de la force de la contraction musculaire que pendant une période de travail (force de contraction établie en plateau) , et d'interdire ladite augmentation pendant un temps d'établissement et en l'absence de stimulation, par exemple pendant une période de repos entre deux contractions consécutives.

Les moyens générateurs du stimulateur incluent des moyens de contrôler l'application et le déroulement fidèles du traitement par le patient et d'en écrire les résultats dans lesdits moyens de mémorisation.

Les moyens de programmation incluent des moyens d'interpréter, d'afficher sur un écran, d'éditer et d'imprimer, conformément à un programme et en langage clair, tant les données saisies par le clavier attribué auxdits moyens de programmation que les données mémorisées par lesdits moyens de mémorisation branchés de manière amovible auxdits moyens de programmation.

Les moyens de programmation peuvent être connectés de manière amovible aux moyens générateurs du stimulateur par une jonction série qui assure une fonction on-line desdits moyens de programmation avec lesdits moyens générateurs, permettant ainsi d'ajuster les paramètres d'excitation en fonction directe des réactions immédiates du patient à la stimulation provoquée.

Il est possible de produire une stimulation neuro-musculaire électrique régie par une programmation libre, déterminée par le thérapeute pour définir des schémas séquentiels d'activité musculaire, assurant un traitement optimalement adapté:
- à chaque type de patient concerné;
- aux objectifs thérapeutiques fixés en fonction de chaque cas spécifique;
- à la physiologie moderne du muscle;
- à l'exclusion d'effets secondaires sensitifs et/ou physiologiques nocifs.

Le traitement étant programmé, il est possible au patient de poursuivre l'application du traitement de façon autonome, à domicile ou en milieu hospitalier.

Dans un but de totale sécurité, l'accès aux paramètres programmables et à l'établissement du programme du traitement sont strictement réservés au seul thérapeute, à l'exclusion du patient qui ne peut, lui, agir que sur le réglage manuel de l'intensité du signal impulsionnel délivré à la sortie, dans des limites fixées par le programme.

Tout programme établi pour un traitement donné étant mémorisé de manière amovible, non volatile et transportable, ledit programme peut être fidèlement reproduit en tout temps et en tout lieu au moyen dudit dispositif de stimulation, sans nécessiter de nouvelle programmation.

Ledits programmes étant mémorisés de manière amovible, non volatile et transportable, ils peuvent être interchangés instantanément en toute simplicité, pour permettre des traitements variés préprogrammés.

Ledit programme comprend sur le même support physique une identification de ses caractéristiques spécifiques en langage clair, ce qui permet d'exclure toute confusion quant au contenu dudit programme et au patient.

**Revendications**

1. Dispositif de stimulation neuro-musculaire électrique pour provoquer des contractions et un exercice ou un entraînement musculaires, comprenant au moins un élément de stimulation (B) portable, autonome, commandé par un support d'informations (6) amovible et interchangeable préalablement programmé en fonction du traitement de chaque patient considéré, par un thérapeute au moyen d'un élément programmeur (A), pour fournir à sa sortie, qui est munie d'au moins une paire d'électrodes (34) prévues pour être appliquées sur la partie à traiter du corps du patient, des trains d'impulsions électriques de stimulation, caractérisé en ce que l'élément de stimulation (B) comprend des moyens pour générer des trains d'impulsions électriques dont les caractéristiques sont adaptées aux fibres musculaires lentes, et d'autres trains d'impulsions électriques dont les caractéristiques sont adaptées aux deux types connus de fibres rapides, en ce que cet élément de stimulation comprend des moyens (9) pour assurer, sous la commande du programme séquentiel enregistré sur le support d'informations amovible (6), un traitement du muscle par des trains d'impulsions électriques des deux sortes mentionnées, des moyens étant prévus pour régler la fréquence et la durée des impulsions de trains d'impulsions, ces deux sortes de trains en fonction du cas du patient, en ce que l'élément de stimulation (B) comprend des moyens asservis à un micro-processeur, actionnables par le patient, pour régler exclusivement l'intensité de la stimulation dans les limites programmées, et des moyens de sécurité, incorporés dans le même micro-processeur, imposant une loi de croissance fixée d'avance de l'intensité des courants de stimulation, et pour empêcher une augmentation de l'intensité des courants de stimulation de se produire en dehors d'une période de plateau (b) de la phase de travail de contraction des muscles, et des moyens pour imposer entre les trains d'impulsions successifs prévus pour les fibres rapides au moins, un temps suffisant (c) pour assurer l'élimination des effets nocifs du travail de ces fibres, afin de permettre un traitement de plusieurs heures sans inconvénients.

2. Dispositif selon la revendication 1, caractérisé en ce que l'élément de stimulation comporte des moyens pour enregistrer sur le support d'informations (6) qui commande son fonctionnement pendant le traitement, les indications relatives à la nature, la durée et le nombre des traitements réellement effectués par le patient, pour ensuite permettre au thérapeute de vérifier si le traitement a été exécuté conformément à ses instructions, et en ce que les moyens de réglage prévus sur l'élément de stimulation et à disposition du patient sont limités au réglage manuel de l'intensité des impulsions de sortie, dans les limites fixées par le programme enregistré sur le support de données.

3. Dispositif selon la revendication 1, comprenant aussi un élément programmeur (A) pour enregistrer des programmes sur des supports d'informations destinés à l'élément de stimulation (B) et des moyens pour interconnecter de façon détachable cet élément à l'élément de stimulation, caractérisé en ce qu'il comporte des moyens (22) pour que, pendant la programmation, l'élément de stimulation (B) étant interconnecté avec l'élément programmeur (A), le patient soit lui-même branché sur l'élément programmeur (A), à travers l'élément de stimulation (B), et reçoive les impulsions correspondant au réglage actuel, pour que le thérapeute constate immédiatement sur le patient l'effet de chaque modification du réglage.

4. Dispositif selon la revendication 3, caractérisé en ce que l'élément programmeur (A) comprend un clavier de programmation, des moyens d'affichage commandés par clavier, une imprimante, un micro-processeur, une mémoire et des moyens pour interconnecter de façon détachable le support d'informations (6), pour d'une part, permettre la lecture, la mémorisation et l'interprétation dans un premier temps de programmes standards et/ou d'informations thérapeutiques supportés par un premier support d'informations (6), dit de référence et constitutif d'une bibliothèque et, d'autre part, dans un second temps, après connection d'un second support d'informations (6), destiné lui à la commande du fonctionnement de l'élément de stimulation (B), permettre au thérapeute de composer et d'enregistrer sur ledit support un programme résultant des informations fournies par le support de référence et/ou d'une programmation libre, d'en vérifier l'exactitude au fur et à mesure de la programmation et d'obtenir une version écrite en clair du programme enregistré sur le support d'informations.

**Claims**

1. A device producing electrical neuromuscular stimulation for causing muscle contractions and muscle exercise or training, comprising at least one portable, autonomous stimulating unit (B) controlled by a removable and interchangeable information storage medium (6) which has been programmed beforehand, in accordance with the treatment for each patient in question, by a therapeutist using a programming unit (A), in order to produce trains of stimulating electrical pulses at its output, which is provided with at least one pair of electrodes (34) to be applied to that part of the patient's body which is to be treated, characterized in that the stimulating unit (B) comprises means for generating trains of electrical pulses whose characteristics are adapted to the slow muscle fibres, and other trains of electrical pulses whose characteristics are adapted to the two known types of fast fibres, in that this stimulating unit comprises means (9) for treating the muscle, under the control of the sequential program recorded on the removable information storage medium (6), with trains of electrical pulses of the two kinds mentioned, means being provided for adjusting the frequency and duration of the pulses of these two kinds of pulse trains according to the patient's case, and in that the stimulating unit (B) comprises means, under the control of a microprocessor and capable of being operated by the patient, for adjusting exclusively the intensity of the stimulation within the programmed limits, and safety means, incorporated in the same microprocessor, for imposing a preset growth law governing the intensity of the stimulating currents, and for preventing the intensity of the stimulating currents from increasing outside a plateau period (b) of the contraction work phase of the muscles, and means for imposing, between the successive pulse trains intended at least for the fast fibres, a sufficient time (c) to ensure elimination of the harmful effects of the work of these fibres, so that a treatment can be carried out for several hours without disadvantages.

2. A device according to Claim 1, characterized in that the stimulating unit has means for recording, on the information storage medium (6) which controls its operation during the treatment, the indications relating to the nature, duration and number of the treatments actually carried out by the patient, in order subsequently to enable the therapeutist to check whether the treatment has been executed in accordance with his instructions, and in that the means of adjustment provided on the stimulating unit and accessible to the patient are limited to manual adjustment of the intensity of the output pulses within the limits set by the program recorded on the data storage medium.

3. A device according to Claim 1, also comprising a programming unit (A) for recording programs on information storage media intended for the stimulating unit (B), and means for detachably interconnecting this unit with the stimulating unit, characterized in that it has means (22) so that, during programming, with the stimulating unit (B) interconnected with the programming unit (A), the patient is himself connected to the programming unit (A), via the stimulating unit (B), and receives the pulses corresponding to the present setting, so that the therapeutist immediately observes the effect on the patient of each change of setting.

4. A device according to Claim 3, characterized in that the programming unit (A) comprises a programming keyboard, keyboard-controlled display means, a printer, a microprocessor, a memory and means for detachably interconnecting the information storage medium (6), on the one hand so as to permit, in a first stage, the reading, storage and interpretation of standard programs and/or therapeutic information carried by a first information storage medium (6), called a reference medium and forming part of a library, and on the other hand, in a second stage, after the connection of a second information storage medium (6), the purpose of which is to control the operation of the stimulating unit (B), to enable the therapeutist to configure and record on said medium a program resulting from the information provided by the reference medium and/or from free programming, check its accuracy as programming proceeds and obtain a version, written in plain language, of the program recorded on the information storage medium.

## Patentansprüche

1. Vorrichtung zur neuro-muskulären, elektrischen Stimulierung, um Kontraktionen und ein Üben oder Trainieren von Muskeln zu bewirken, bestehend aus mindestens einem tragbaren, autonomen Stimulierelement (B), gesteuert von einem herausnehmbaren und austauschbaren Informationsträger (6), der vorher in Abhängigkeit vor der Art der Behandlung des zu behandelnden Patienten programmiert wurde durch einen Therapeuten mit Hilfe eines Programmierelementes (A), um an seinem Ausgang, der mit mindestens ein Paar Elektroden (34) ausgerüstet ist, das dazu bestimmt ist an die zu behandelnde Körperpartie des Patienten angelegt zu werden, elektrische Stimulier-Impulsfolgen abzugeben, dadurch gekennzeichnet, daß das Stimulierelement (B) Mittel zur Erzeugung der elektrischen Impulssequenzen enthält, deren Charakteristiken an die langsamen Muskelfasern angepaßt sind, und andere elektrische Impulsfolgen, deren Charakteristiken an zwei bekannte Arten schneller Muskelfasern angepaßt sind, sowie dadurch, daß dieses Stimulierelement Mittel (9) enthält, um, gesteuert durch das auf dem herausnehmbaren Informationsträger (6) aufgezeichnete, sequentielle Programm, eine Behandlung des Muskels durch elektrische Impulsfolgen der beiden erwähnten Arten zu ermöglichen, wobei Mittel vorgesehen sind, um die Frequenz und Dauer der Impulse der Impulsfolgen und diese beiden Arten von Folgen in Abhängigkeit von der Art des Patienten einzustellen, und wobei des weiteren das Stimulierelement (B) mikroprozessorunterstützte, vom Patienten bedienbare Mittel ausschließlich zur Einstellung der Intensität der Stimulierung in den programmierten Grenzen enthält, und außerdem im gleichen Mikroprozessor eingebaute Sicherheitsmittel, durch die ein vorher festgelegtes Zunahmegesetz für die Intensität der Stimulierströme aufgedrückt wird, und die verhindern, daß außerhalb einer Plateauperiode (b) der Kontraktionsarbeitsphase der Muskeln eine Zunahme der Intensität der Stimulierströme erfolgt, und schließlich Mittel, durch die zwischen für die schnellen Fasern aufeinanderfolgenden Impulsfolgen mindestens eine ausreichende Zeit (c) eingelegt wird, um die Beseitigung der schädlichen Arbeitseffekte dieser Fasern sicherzustellen, wodurch eine Behandlung von mehreren Stunden ohne Nachteile ermöglicht wird.

2. Vorrichtung nach Patentanspruch 1, dadurch gekennzeichnet, daß das Stimulierelement Elemente enthält, um auf dem Informationsträger (6), der sein Funktionieren während der Behandlung steuert, die Angaben betreffend die Art, die Dauer und die Anzahl der am Patienten durchgeführten Behandlungen aufzuzeichnen, so daß es dadurch dem Therapeuten ermöglicht wird zu kontrollieren, daß die Behandlung gemäß seinen Anweisungen ausgeführt wurde, und dadurch gekennzeichnet, daß die am Stimulierelement vorgesehenen und dem Patienten zur Verfügung stehenden Mittel auf die manuelle Einstellung der Ausgangsimpulse innerhalb der auf dem Datenträger aufgezeichneten Programmgrenzen beschränkt sind.

3. Vorrichtung nach Patentanspruch 1, bestehend auch aus einem Programmierelement (A) zur Aufzeichnung der Programme auf den Informationsträgern, die für das Stimulierelement bestimmt sind und aus Mitteln zum trennbaren Verbinden dieses Elementes mit dem Stimulierelement, dadurch gekennzeichnet, daß es Mittel (22) enthält, damit während des Programmierens, während dem das Stimulierelement (B) mit dem Programmierelement (A) verbunden ist, der Patient selbst an das Programmierelement (A) über das Stimulierelement (B) angeschaltet ist und die Impulse entsprechend der aktuellen Einstellung empfängt, damit der Therapeut sofort die Auswirkungen aller Veränderungen der Einstellung feststellen kann.

4. Vorrichtung nach Patentanspruch 3, dadurch gekennzeichnet, daß das Programmierelement (A) eine Programmiertastatur, von der Tastatur gesteuerte Anzeigemittel, einen Drucker, einen Mikroprozessor, einen Speicher und Mittel zur trennbaren Verbindung mit dem Informationsträger (6) enthält, um einerseits das Lesen, das Speichern und die Interpretation in einer ersten Standardprogrammierzeit und/oder therapeutische Informationszeit zu ermöglichen, gespeichert auf einem ersten Informationsträger (6), genannt Referenzinformationsträger und Bestandteil einer Bibliothek, und um, andererseits, in einer zweiten Zeit, nach Verbinden mit einem zweiten Informationsträger (6), der zum Steuern des Funktionierens des Stimulierelements (B) bestimmt ist, dem Therapeuten zu ermöglichen auf dem Träger ein Programm zusammenzusetzen und aufzuzeichnen, das sich aus den Informationen ergibt, die vom Referenzträger und/oder durch eine freie Programmierung geliefert wurden, um die Genauigkeit der Programmierung daraus zu überprüfen und um eine schriftliche Version des auf dem Informationsträger aufgezeichneten Programms in Klartext zu erhalten.

# FIG.1

FIG.2

FIG.3

FIG.4

FIG.5

FIG.6

EP 0 197 889 B1

**FIG.7a**

**FIG.7b**

**FIG.7c**

**FIG.8a**

**FIG.8b**

EP 0 197 889 B1

# FIG.9a

# FIG.9b

# FIG.10